# EUROPEAN PATENT SPECIFICATION

(11) **EP 0 705 095 B1**
(45) Date of publication and mention of the grant of the patent: **26.11.1997**
(21) Application number: 93915325.0
(22) Date of filing: 11.06.1993
(51) Int. Cl.: A61K 9/00

(54) **COMBINATIONS OF VISCOELASTICS FOR USE DURING SURGERY**
VERWENDUNG EINER KOMBINATION AUS VISCOELASTISCHEN MITTELN WÄHREND DER CHIRUGIE
COMBINAISONS DE VISCO-ELASTIQUES A UTILISER PENDANT DES INTERVENTIONS CHIRURGICALES

(30) Priority: 12.06.1992 US 897733
(43) Date of publication of application: 10.04.1996
(73) Proprietor: ALCON LABORATORIES, INC., Fort Worth Texas 76134-2099 (US)
(72) Inventor: McLAUGHLIN, Richard, N., Arlington, TX 76017 (US); LORENZETTI, Ole, J., Fort Worth, TX 76110 (US)
(74) Representative: Hanna, Peter William Derek
(86) International application number: PCT/US93/05639
(87) International publication number: WO 93/25187

(56) References cited:
- EP-A- 0 136 782
- EP-A- 0 244 178
- WO-A-89/03205
- US-A- 4 141 973
- US-A- 4 819 617

## Description

### Background of the Invention

There are known viscous or viscoelastic agents for ophthalmic surgical use (hereinafter "agent" or "viscoelastic agent"), for example, the VISCOAT® product (Alcon Surgical, Inc.), which contains sodium hyaluronate and chondroitin sulfate; Healon® and Healon® GV (Kabi Pharmacia), Amvisc® and Amvisc Plus® (IOLAB), and Vitrax® (Allergan), all of which contain essentially pure sodium hyaluronate; and Occucoat® (Storz), which contains hydroxypropylmethylcellulose (HPMC). All of these highly purified products are useful in certain ocular surgical procedures, such as cataract surgery. They are used by the skilled ophthalmic surgeon for several surgical purposes, including maintenance of intraocular spaces, protection of ophthalmic tissues, particularly corneal endothelial cells, and as an aid in manipulating ophthalmic tissues. These agents are generally viscous enough to permit the skilled surgeon to use them for their intended surgical purposes, but not so viscous that expression of the agent through a cannula of acceptable bore size might be made difficult.

Combinations of viscoelastic agents are described in the patent literature, i.e. where the components are mixed together and presented as an admixed composition. For example, EP-0,136,782 (Cilco Inc.) discloses the combination of chondroitin sulfate and hyaluronate, US-4,819,617 (Goldberg) discloses combinations of carboxymethyl cellulose and hyaluronic acid or chondroitin sulfate, WO-89/03205 (University of Florida) discloses combinations of polyvinyl pyrrolidone with hyaluronic acid, chondroitin sulfate or carboxymethyl cellulose, and EP-0,244,178 (Iolab Inc.) discloses a polysaccharide composition comprising a mixture of hyaluronic acid and chondroitin sulfate.

There is, however, no one viscoelastic agent which best fulfils all of the surgical purposes. Due to their particular physical characteristics, certain viscoelastic agents will be better suited for particular aspects of the surgical procedure. For example, in cataract surgery, the combination of relatively low molecular weight sodium hyaluronate and chondroitin sulfate found in the VISCOAT® product works extremely well in maintaining the anterior chamber during capsulotomy, or anytime during the cataract procedure, and in adhering to and protecting tissues, particularly the cornea endothelium. However, due to its adhering and coating characteristics, the VISCOAT® product is more difficult to remove from the anterior chamber of the eye than some other agents. In addition, although it can be used to manipulate tissue for insertion of an intraocular lens (IOL) into the eye, certain other agents are known to perform this function better.

Viscoelastic solutions of relatively high molecular weight sodium hyaluronate having functionally desirable viscosity, such as Healon® or the PROVISC™ product (Alcon Laboratories, Inc.), are highly cohesive, but relatively non-adherent with respect to the tissues they may contact during surgery. These characteristics make such solutions well suited for use as a soft tool for the gentle manipulation of delicate tissues during surgery. For example, these viscoelastic agents can be used to inflate the capsular bag and facilitate the insertion of an IOL. Their cohesiveness and lack of adhesiveness also make them easier to remove from the eye at the end of surgery. However, sodium hyaluronate is not as effective as some agents in protecting ophthalmic tissues, especially during phacoemulsification procedures.

HPMC adheres well to ophthalmic tissues and therefore protects them, but does not perform as well as, for example, the VISCOAT® product, in maintaining the anterior chamber, or as well as sodium hyaluronate in manipulating tissues. However, it can be easily diluted with irrigation fluid following IOL implantation. The removal of the viscous or viscoelastic agent at the close of surgery may help to prevent intraocular pressure spikes following surgery.

In general, viscous solutions containing relatively higher molecular weight agents, including high molecular weight sodium hyaluronate, are more effective in maintaining the intraocular space than less viscous solutions containing relatively lower molecular weight agents; however, the high molecular weight agents tend to be highly cohesive and may be prematurely aspirated from a surgical site. This may occur, for instance, if they come into contact with the phacoemulsification tip during a phacoemulsification procedure. The relatively lower molecular weight products, which due to their tenacious characteristics adhere to and protect tissues, are more difficult to remove from the surgical site.

It would be advantageous to use a system containing more than one viscoelastic agent during an ophthalmic procedure, such as a cataract operation, to obtain the maximum benefits offered by the variety of available viscoelastic agents. The present invention provides this advantage.

### Summary of the Invention

The present invention is directed to use of a first and second viscoelastic agent in the manufacture of a packaged product, in accordance with claim 1. The product is employed during ophthalmic surgery in humans in order to achieve satisfactory intraocular space maintenance and ocular tissue protection, and to provide for manipulation of ocular tissues and ease of removal of the viscoelastic agents at the end of the procedure. An object of the invention is to provide a packaged product comprising viscoelastic agents possessing different cohesive or adherent properties. This enables the skilled surgeon to use viscoelastic agents exhibiting a relatively greater degree of cohesiveness for certain steps of a surgical procedure, and to use a more adherent viscoelastic agent for other steps in the same procedure. The surgeon is thereby afforded the benefit of the use of multiple viscoelastic agents, each of which is particularly well suited for certain steps of the procedure.

### Detailed Description of the Invention

The present invention comprises use of multiple viscoelastic agents having different adherent or cohesive properties, which multiple agents are separately maintained for use in a single surgical procedure. Those skilled in the art will recognize that the systems of the present invention can be employed by the skilled surgeon in a variety of surgical procedures that may be improved by alternately choosing agents with the desired characteristics to either help manipulate tissues or to function as an adherent protective agent. The preferred product is useful in ophthalmic surgery.

Viscoelastic agents which are useful for use in the present invention include but are not limited to: sodium hyaluronate, chondroitin sulfate, polyacrylamide, HPMC, proteoglycans, collagen, methylcellulose, carboxymethyl cellulose, ethylcellulose, polyvinylpyrrolidone and keratan, all of various molecular weights and concentrations, or combinations thereof. Those skilled in the art will appreciate that the suitability of a given agent for a particular step in a surgical procedure will depend upon such things as the agent's concentration, average molecular weight, viscosity, pseudoplasticity, elasticity, rigidity, adherence (coatability), cohesiveness, molecular charge, and osmolality in solution. The agent's suitability will depend further on the function(s) which the agent is expected to perform and the surgical technique being employed by the surgeon.

For portions of surgical procedures involving phacoemulsification and/or irrigation/aspiration, it is generally preferable to use a viscoelastic agent that possesses relatively greater adherent properties and relatively lesser cohesive properties. Such viscoelastic agents are referred to herein as "adherent" agents. The cohesiveness of a viscoelastic agent in solution is thought to be dependent, at least in part, on the average molecular weight of that agent. At a given concentration, the greater the molecular weight, the greater the cohesiveness. The adherent agents, which are relatively lacking in cohesiveness, therefore will typically be of lower molecular weight; the molecular weight will typically be less than 1,000,000 daltons, preferably less than 750,000 daltons. To achieve a functionally desirable viscosity, the concentrations of the lower molecular weight agents in solution will need to be relatively higher than for higher molecular weight agents. These concentrations will typically be at least about 2% weight to volume (e.g. Occucoat®). The VISCOAT® product, for example, contains approximately 4% chondroitin sulfate (∼ 25,000 daltons) and 3% sodium hyaluronate (∼ 700,000 daltons). Vitrax® is believed to contain approximately 3% sodium hyaluronate (∼ 500,000 daltons). For agents such as these, which are being employed primarily for protective purposes as opposed to tissue manipulation purposes, a functionally desirable viscosity will be a viscosity sufficient to permit a protective layer of such agent to remain on the tissue or cells of concern during the surgical step(s) being performed. Such viscosity will typically be from about 3,000 mPas (cps) to about 60,000 mPas (cps) (at shear rate of 2 sec⁻¹ and 25° C), and preferably will be about 40,000 mPas (cps). Such adherent agents are capable of providing the protective function previously discussed, yet are not prone to inadvertent removal, which could jeopardize the delicate tissue being protected.

Those portions of surgical procedures involving manipulation of delicate tissue are generally better served by viscoelastic agents that possess relatively greater cohesive properties and relatively lesser adherent properties. Such agents are referred to herein as "cohesive" agents. Typically, these cohesive agents will possess average molecular weights in excess of 1,000,000 daltons and will have functionally desirable viscosity at concentrations of not more than about 1.6% weight to volume. Examples of such cohesive agents are: the PROVISC™ product, Healon®, Healon® GV, Amvisc® and Amvisc Plus®. For cohesive agents such as these, which are being employed primarily for tissue manipulation or maintenance purposes as opposed to protective purposes, a functionally desirable viscosity will be a viscosity sufficient to permit the skilled surgeon to use such agent as a soft tool to manipulate or support the tissue of concern during the surgical step(s) being performed. Such viscosity will depend upon the average molecular weight of the agent and its concentration in solution. Most preferred are cohesive agents having an average molecular weight of at least about 2,000,000 daltons and a concentration in solution of between about 1.0 to about 1.4% weight to volume. Such cohesive agents are capable of maintaining intraocular space and manipulating tissue without adhering to it. When their purpose has been served, they can, because of their cohesive properties, be readily removed with minimal trauma to the surrounding tissue.

In a preferred system for cataract surgery, one viscoelastic agent is used during capsulotomy, expression or phacoemulsification of the cataractous lens, and irrigation/aspiration (Stage 1), and a different viscoelastic agent is used following extraction of the lens and during implantation of an IOL (Stage 2). The agent used during Stage 1 of the surgery should be adherent enough to be retained in the anterior chamber so as to protect the delicate endothelial cells. Preferably, a sufficient volume of the solution containing the Stage 1 agent should be available to fill and maintain anterior chamber space. Approximately 0.3 to 0.5 mL is typically utilized for this purpose. Most preferably, the solution of the Stage 1 agent should exhibit sufficient viscosity to at least partially relieve lens convexity, i.e., flatten the lens somewhat so that a capsulotomy can be performed with more control and less chance of peripheral capsular tearing. The primary purpose of the adherent agent is to protect the tissues, particularly the corneal endothelial cells, from trauma resulting from shear forces and direct contact from instruments during the capsulotomy and from nuclear fragments during removal of the cataractous lens.

The cohesive agent, used during Stage 2, should effectively allow for implantation of an IOL by facilitating the manipulation of tissue, i.e., filling and opening the capsular bag in which the IOL will be placed, and should also maintain the anterior chamber prior to and during implantation of the IOL. It should also be relatively easy to remove from the eye after IOL implantation.

Thus, a packaged product manufactured in accordance with the invention, as preferably used in cataract surgery, may for example be used in a procedure consisting of the following steps: surgically opening the eye; filling the anterior chamber with an adherent agent for use in Stage 1; performing a capsulotomy; removing any cataractous tissue; filling the capsular bag with a cohesive agent for use in Stage 2; and implanting an IOL in the capsular bag. The skilled surgeon will generally remove all or part of the cohesive agent, the adherent agent, or both, subsequent to implantation of the IOL in the capsular bag.

The preferred system for cataract surgery comprises an adherent agent containing sodium hyaluronate and chondroitin sulfate for use during Stage 1 of a procedure, and a separately maintained cohesive agent containing a relatively high molecular weight sodium hyaluronate agent for use during Stage 2. Most preferably the adherent agent utilized during Stage 1 agent will contain approximately 4% chondroitin sulfate (∼ 25,000 daltons) and 3% sodium hyaluronate (∼ 700,000 daltons) and will have a viscosity of approximately 40,000 cps (at a shear rate of 2 sec⁻¹ and 25° C) as found in the VISCOAT® product. The most preferred adherent agent for use during Stage 2 will consist of essentially pure sodium hyaluronate having an average molecular weight greater than about 2,000,000 daltons and a concentration of about 1.0% to about 1.4% weight to volume, as found in the PROVISC™ product, Healon® and Healon® GV. The Stage 1 adherent agent is to be used upon the surgeon's entrance into the anterior chamber. The purpose of this agent is to fill and maintain the chamber and protect the tissues during capsulotomy, phacoemulsification and/or irrigation/aspiration and removal of the cataractous lens elements. The Stage 2 cohesive agent is then introduced into the empty capsular bag to inflate it for introduction and placement of an IOL. During its introduction, the high molecular weight sodium hyaluronate may partially displace the VISCOAT® product and in that manner supplement the maintenance of the corneal dome. Alternatively, some of the VISCOAT® product may be removed by the surgeon prior to the introduction of the Stage 2 cohesive agent. Upon completion of IOL placement the Stage 2 agent can be removed readily with irrigation/aspiration techniques known to those skilled in the art. Such removal may help prevent a sharp post surgical increase in intraocular pressure. The use of these two agents during cataract surgery provides for optimal maintenance of the anterior chamber, protection of tissues, and manipulation of the capsular bag for IOL implantation.

Packaged products manufactured in accordance with the present invention may also be used in corneal transplant surgery. In conjunction with the removal of the patient's cornea button, it is desirable to replace the aqueous humor with a highly viscous agent that will provide a firm bed to support the donor cornea, yet be susceptible to easy removal upon completion of the surgery. The donor graft, on the other hand, requires maximum protection from the surgical trauma and should therefore be coated with a different more adherent agent. Thus, such products may be utilized in corneal transplant surgery in the following manner. Upon removing the patient's corneal button, the aqueous humor is replaced, in total or in part, with a highly viscous, highly cohesive agent, such as the high molecular weight sodium hyaluronate of the PROVISC™ product or Healon® GV. The donor graft is carefully removed from its storage medium and coated with an adherent agent, such as the VISCOAT® product or HPMC. In addition, the contact portions of any instrument used to handle the donor graft may be coated with the adherent agent prior to such handling. The donor cornea is then placed on the bed created by the cohesive agent, and sutured into place. The cohesive agent may be evacuated from the eye just prior to completion of the transplant, or immediately thereafter by known methods.

Packaged products manufactured in accordance with the present invention may also be used in posterior segment surgery. In a retinal detachment procedure, for example, a highly viscous, cohesive agent such as the PROVISC™ product or Healon® GV will be used to manipulate the retina into position against the basement membrane of the choroid. Small amounts of an adherent agent, such as the VISCOAT® product, may be injected behind the retina before or after such manipulation to temporarily maintain the contact between the retina and basement membrane while more permanent attachment procedures well known to those skilled in the art are performed (e.g. tacking or laser welding).

It will be appreciated that the different viscoelastic agents for use in the present invention have to be maintained in separate or common containers. Preferably, there are two such agents, which are conventionally packaged together. In a preferred embodiment, an adherent agent, such as the VISCOAT® product, and a cohesive agent, such as the PROVISC™ product, will be packaged together in a single, sterile package for the surgeon's convenience. In such a common package, the agents may be contained in separate vials or syringes, or loaded into a common vial or syringe. In either configuration, it may be desirable to use one or more agent that has been stained with a pharmacologically acceptable dye or otherwise marked, so that the different agents may be more readily distinguished by the surgeon during use.

Neither the type of packaging nor the specific means by which the agents are administered are critical to this invention, the scope of which is intended to include any assortment, collection, kit or product which permits alternate or subsequent separate administration to the surgical site.

## Claims

1. Use of a first and second viscoelastic agent in the manufacture of a packaged product comprising said first viscoelastic agent and said second viscoelastic agent, for performing an ophthalmic surgical procedure in humans, characterised in that said first viscoelastic agent has greater adherent properties than said second viscoelastic agent, and said second viscoelastic agent has greater cohesive properties than said first viscoelastic agent, and wherein the two viscoelastic agents are maintained to permit alternate or subsequent separate administration to the surgical site.

2. Use according to claim 1, wherein the first viscoelastic agent and the second viscoelastic agent are in separate containers within a common package.

3. Use according to claim 1, wherein the first viscoelastic agent and second viscoelastic agent are contained in a single container within a common package.

4. Use according to claim 1, wherein at least one of the first viscoelastic agent and second viscoelastic agent is marked to permit visual differentiation by the user of the product.

5. Use according to claim 4, wherein at least one of the first viscoelastic agent and second viscoelastic agent is marked with a pharmacologically acceptable dye.

6. Use according to claim 1, wherein the first and second viscoelastic agents are independently selected from the group consisting of sodium hyaluronate, chondroitin sulfate, polyacrylamide, collagen, methylcellulose, ethylcellulose, hydroxypropylmethylcellulose, carboxymethylcellulose, polyvinyl pyrrolidone, keratan, and combinations thereof.

7. Use according to claim 6, wherein the first viscoelastic agent has a viscosity from about 3,000 to about 60,000 mPas (cps).

8. Use according to claim 7, wherein the first viscoelastic agent comprises a combination of sodium hyaluronate and chondroitin sulfate.

9. Use according to claim 8, wherein the viscosity of the first viscoelastic agent is about 40,000 mPas (cps).

10. Use according to claim 9, wherein the second viscoelastic agent has an average molecular weight of greater than about 1,000,000 daltons.

11. Use according to claim 10, wherein the second viscoelastic agent comprises sodium hyaluronate.

12. Use according to claim 11, wherein the average molecular weight of the second viscoelastic agent is greater than about 2,000,000 daltons.

13. Use according to claim 6, wherein the first viscoelastic agent comprises a combination of sodium hyaluronate at a concentration of approximately 3% weight to volume and chondroitin sulfate at a concentration of approximately 4% weight to volume, and wherein the second viscoelastic agent comprises sodium hyaluronate at a concentration of approximately 1.0% weight to volume.

14. Use according to claim 13, wherein the sodium hyaluronate of the first viscoelastic agent has an average molecular weight of less than 1,000,000 daltons, the chondroitin sulfate of the first viscoelastic agent has a molecular weight of about 25,000 daltons, and the sodium hyaluronate of the second viscoelastic agent has an average molecular weight of greater than 2,000,000 daltons.

## Patentansprüche

1. Verwendung eines ersten und zweiten viskoelastischen Agens bei der Herstellung eines abgepackten Produktes, das besagtes erste viskoelastische Agens und besagtes zweite viskoelastische Agens umfaßt, zur Durchführung eines ophthalmischen chirurgischen Verfahrens bei Menschen, dadurch gekennzeichnet, daß besagtes erste viskoelastische Agens höhere Adhäsionseigenschaften besitzt als besagtes zweite viskoelastische Agens und daß besagtes zweite viskoelastische Agens höhere Kohäsionseigenschaften besitzt als besagtes erste viskoelastische Agens und wobei die zwei viskoelastischen Agentien so gehalten werden, daß alternierende oder aufeinanderfolgende getrennte Applikation auf die Operationsstelle ermöglicht wird.

2. Verwendung nach Anspruch 1, wobei das erste viskoelastische Agens und das zweite viskoelastische Agens sich in getrennten Behältern in einer gemeinsamen Verpackung befinden.

3. Verwendung nach Anspruch 1, wobei das erste viskoelastische Agens und das zweite viskoelastische Agens in einem einzigen Behälter in einer gemeinsamen Verpackung enthalten sind.

4. Verwendung nach Anspruch 1, wobei wenigstens eines von dem ersten viskoelastischen Agens und zweiten viskoelastischen Agens markiert ist, um visuelle Unterscheidung durch den Benutzer des Produktes zu ermöglichen.

5. Verwendung nach Anspruch 4, wobei wenigstens eines von dem ersten viskoelastischen Agens und zweiten viskoelastischen Agens mit einem pharmakologisch annehmbaren Farbstoff markiert ist.

6. Verwendung nach Anspruch 1, wobei die ersten und zweiten viskoelastischen Agentien unabhängig ausgewählt werden aus der Gruppe, die aus Natriumhyaluronat, Chrondroitinsulfat, Polyacrylamid, Kollagen, Methylcellulose, Ethylcellulose, Hydroxypropylmethylcellulose, Carboxymethylcellulose, Polyvinylpyrrolidon, Keratan und Kombinationen derselben besteht.

7. Verwendung nach Anspruch 6, wobei das erste viskoelastische Agens eine Viskosität von etwa 3.000 bis etwa 60.000 mPas (cps) besitzt.

8. Verwendung nach Anspruch 7, wobei das erste viskoelastische Agens eine Kombination aus Natriumhyaluronat und Chrondroitinsulfat umfaßt.

9. Verwendung nach Anspruch 8, wobei die Viskosität des ersten viskoelastischen Agens etwa 40.000 mPas (cps) beträgt.

10. Verwendung nach Anspruch 9, wobei das zweite viskoelastische Agens ein mittleres Molekulargewicht von mehr als etwa 1.000.000 Daltons besitzt.

11. Verwendung nach Anspruch 10, wobei das zweite viskoelastische Agens Natriumhyaluronat umfaßt.

12. Verwendung nach Anspruch 11, wobei das mittlere Molekulargewicht des zweiten viskoelastischen Agens höher ist als etwa 2.000.000 Daltons.

13. Verwendung nach Anspruch 6, wobei das erste viskoelastische Agents eine Kombination aus Natriumhyaluronat in einer Konzentration von ungefähr 3%, Gewicht zu Volumen, und Chondroitinsulfat in einer Konzentration von ungefähr 4%, Gewicht zu Volumen, umfaßt, wobei das zweite viskoelastische Agens Natriumhyaluronat in einer Konzentration von ungefähr 1,0%, Gewicht zu Volumen, umfaßt.

14. Verwendung nach Anspruch 13, wobei das Natriumhyaluronat des ersten viskoelastischen Agens ein mittleres Molekulargewicht von weniger als 1.000.000 Daltons besitzt, das Chondroitinsulfat des ersten viskoelastischen Agents ein Molekulargewicht von etwa 25.000 Daltons besitzt und das Natriumhyaluronat des zweiten viskoelastischen Agents ein mittleres Molekulargewicht von mehr als 2.000.000 Daltons besitzt.

## Revendications

1. Utilisation d'un premier agent visco-élastique et d'un second agent visco-élastique dans la fabrication d'un produit emballé comprenant ledit premier agent visco-élastique et ledit second agent visco-élastique, pour la mise en oeuvre d'une opération de chirurgie ophtalmologique chez l'homme, caractérisée en ce que ledit premier agent visco-élastique présente des propriétés d'adhérence supérieures à celles dudit second agent visco-élastique, et ledit second agent visco-élastique présente des propriétés de cohésion supérieures à celles dudit premier agent visco-élastique, et dans laquelle les deux agents visco-élastiques sont maintenus pour pouvoir être administrés de manière alternée ou bien séparément de manière successive.

2. Utilisation suivant la revendication 1, dans laquelle le premier agent visco-élastique et le second agent visco-élastique sont dans des récipients distincts à l'intérieur d'un emballage commun.

3. Utilisation suivant la revendication 1, dans laquelle le premier agent visco-élastique et le second agent visco-élastique sont présents dans un seul récipient à l'intérieur d'un emballage commun.

4. Utilisation suivant la revendication 1, dans laquelle au moins un des agents consistant en le premier agent visco-élastique et le second agent visco-élastique est marqué pour permettre une différenciation visuelle par l'utilisateur du produit.

5. Utilisation suivant la revendication 4, dans laquelle au moins un des agents consistant en le premier agent visco-élastique et le second agent visco-élastique est marqué avec un colorant pharmacologiquement acceptable.

6. Utilisation suivant la revendication 1, dans laquelle les premier et second agents visco-élastiques sont choisis, indépendamment, dans le groupe consistant en hyaluronate de sodium, chondroïtine-sulfate, polyacrylamide, collagène, méthylcellulose, éthylcellulose, hydroxypropylméthylcellulose, carboxyméthylcellulose, polyvinylpyrrolidone, kératane et leurs associations.

7. Utilisation suivant la revendication 6, dans laquelle le premier agent visco-élastique a une viscosité d'environ 3000 à environ 60 000 mPas (cps).

8. Utilisation suivant la revendication 7, dans laquelle le premier agent visco-élastique comprend une association de hyaluronate de sodium et de chondroïtine-sulfate.

9. Utilisation suivant la revendication 8, dans laquelle la viscosité du premier agent visco-élastique est égale à environ 40 000 mPas (cps).

10. Utilisation suivant la revendication 9, dans laquelle le second agent visco-élastique a un poids moléculaire moyen supérieur à environ 1 000 000 de daltons.

11. Utilisation suivant la revendication 10, dans laquelle le second agent visco-élastique comprend le hyaluronate de sodium.

12. Utilisation suivant la revendication 11, dans laquelle le poids moléculaire moyen du second agent visco-élastique est supérieur à environ 2 000 000 de daltons.

13. Utilisation suivant la revendication 6, dans laquelle le premier agent visco-élastique comprend une association de hyaluronate de sodium à une concentration d'approximativement 3 % en poids/volume et de chondroïtine-sulfate à une concentration d'approximativement 4 % en poids/volume, et dans laquelle le second agent visco-élastique comprend du hyaluronate de sodium à une concentration d'approximativement 1,0 % en poids/volume.

14. Utilisation suivant la revendication 13, dans laquelle le hyaluronate de sodium du premier agent visco-élastique a un poids moléculaire moyen inférieur à 1 000 000 de daltons, le chondroïtine-sulfate du premier agent visco-élastique a un poids moléculaire d'environ 25 000 daltons, et le hyaluronate de sodium du second agent visco-élastique a un poids moléculaire moyen supérieur à 2 000 000 de daltons.
